(19) Europäisches Patentamt
European Patent Office
Office européen des brevets

(11) **EP 1 788 536 B1**

(12) **FASCICULE DE BREVET EUROPEEN**

(45) Date de publication et mention
de la délivrance du brevet:
**13.02.2008 Bulletin 2008/07**

(51) Int Cl.:
**G08B 21/06** (2006.01)     **A61B 5/18** (2006.01)
**B60K 28/06** (2006.01)

(21) Numéro de dépôt: **06022592.7**

(22) Date de dépôt: **30.10.2006**

(54) **Procédé d'évaluation de l'état de vigilance d'un conducteur de véhicule**

Verfahren zur Bewertung des Aufmerksamkeitszustands eines Fahrzeugfahrers

Method for the evaluation of the state of vigilance of a vehicle conductor

(84) Etats contractants désignés:
**DE ES GB IT**

(30) Priorité: **17.11.2005 FR 0511650**

(43) Date de publication de la demande:
**23.05.2007 Bulletin 2007/21**

(73) Titulaire: **Siemens VDO Automotive**
**31036 Toulouse Cedex 1 (FR)**

(72) Inventeurs:
• **Boverie, Serge**
**31830 Plaisance du Touch (FR)**

• **Giralt, Alain**
**31400 Toulouse (FR)**

(74) Mandataire: **Maier, Daniel Oliver**
**Siemens Aktiengesellschaft**
**Postfach 22 16 34**
**80506 München (DE)**

(56) Documents cités:
**US-A- 6 070 098**

## Description

L'invention concerne un procédé d'évaluation de l'état de vigilance d'un conducteur de véhicule, et vise de façon plus spécifique un procédé d'évaluation basé sur l'analyse des mouvements des paupières du conducteur, permettant de détecter chaque fermeture de paupière, dite clignement, et de fournir une information représentative de la durée du dit clignement.

US 6,070,098 décrit un procédé d'évaluation de l'état de vigilance d'un conducteur de véhicule, consistant à analyser les mouvements d'au moins une paupière du dit conducteur de façon à détecter chaque fermeture de la dite paupière, dite clignement, et à fournir une information représentative de la durée du dit clignement, le dit procédé d'évaluation consistant:

- à établir une classification des durées de clignement, composée de classes adaptées pour présenter des zones frontières de transition progressive définies par mise en oeuvre d'une méthode mathématique telle que la logique "floue" ;
- à établir une classification d'états de vigilance.

Sur la base de ce principe, l'objectif essentiel de l'invention est de fournir un procédé d'évaluation permettant de réaliser en ligne un diagnostic de la baisse de vigilance d'un conducteur à partir d'informations d'ordre physiologique.

Un autre objectif de l'invention est de fournir un procédé d'évaluation conçu pour introduire des niveaux de pondération du diagnostic en fonction d'observations environnementales et comportementales.

A cet effet, l'invention vise, en premier lieu, un procédé d'évaluation consistant :

- dans une phase préalable :

  • à établir une classification des durées de clignement composée de m classes délimitant m plages contiguës de valeurs de durées de clignements, consistant en au moins deux classes correspondant respectivement à des clignements dits de moyenne durée « M », et de longue durée « L », les dites classes étant adaptées pour présenter des zones frontières de transition progressive, définies par exemple par mise en oeuvre d'une méthode mathématique telle que la logique « floue »,

  • et à établir une classification d'états de vigilance composée de n classes d'état de vigilance, avec n ≥ 2, comportant :

    * une classe correspondant à un état « éveillé », définie par un nombre de clignements de moyenne durée inférieur à un seuil donné, et/ou un nombre de clignements de longue durée inférieur à un seuil donné,
    * et une classe correspondant à un état « endormi », définie par un nombre de clignements de moyenne durée supérieur à un seuil donné, et/ou un nombre de clignements de longue durée supérieur à un seuil donné,

- et lors du déroulement d'une procédure d'évaluation :

  • à associer à chaque clignement un vecteur de durée (m, 1) dont chaque composante représente le degré d'appartenance du dit clignement à une des m classes de durée prédéfinies,

  • à définir des fenêtres temporelles d'analyse consistant en des intervalles de temps au terme de chacun desquels on calcule un vecteur cumulé de durée dont chaque composante consiste en la somme $\Sigma M$, $\Sigma L$ des composantes de même ligne des vecteurs de durée correspondant aux clignements détectés pendant la fenêtre d'analyse,

  • et à déduire de la comparaison des valeurs calculées $\Sigma M$ et $\Sigma L$ avec les valeurs seuils correspondantes de la classification des états de vigilance, une information représentative de l'état de vigilance du conducteur.

De façon générale, ce procédé d'évaluation est basé sur l'observation des mouvements des paupières sur un intervalle de temps donné (fenêtre d'analyse), et conduit à une estimation en temps réel et en instantané de l'état de vigilance du conducteur.

Par ailleurs, ce procédé d'évaluation consiste, en premier lieu, à introduire, dans la classification des durées de clignement, un degré de progressivité qui conduit :

- à classer chaque clignement soit dans l'une ou l'autre classe de durée, soit simultanément dans deux classes contiguës avec un degré d'appartenance à chacune des dites classes compris entre 0 et 1, la somme de ces deux degrés d'appartenance étant toujours égale à 1,
- et à définir chaque clignement par un vecteur (m, 1) dont chaque composante correspond à un degré d'appartenance à une classe de durée.

Ce procédé d'évaluation consiste, en outre, à définir une classification des états de vigilance conçue pour permettre de fournir directement une estimation de l'état de vigilance sur une fenêtre d'analyse en fonction du nombre et de la nature des clignements détectés durant cette fenêtre d'analyse.

Il est à noter, à cet effet, que, de façon spécifique selon l'invention, chaque valeur $\Sigma M$, $\Sigma L$ utilisée pour estimer l'état de vigilance consiste en la somme, sur une fenêtre d'analyse, des degrés d'appartenance à une classe de durée donnée, en l'exemple respectivement la classe moyenne durée et la classe longue durée.

Ainsi, par exemple pour la détermination de la valeur $\Sigma M$, un clignement auquel est associé un degré d'appartenance à la classe moyenne durée égal à 1, pèse le même poids que dix clignements dont le degré d'appartenance à la classe moyenne durée est égal à 0,1.

Selon un mode de mise en oeuvre avantageux de l'invention, on déclenche l'ouverture d'une fenêtre d'analyse lors de chaque détection d'un clignement, chaque fenêtre d'analyse ouverte couvrant une période de temps déterminée précédant le dit déclenchement.

Ainsi, les fenêtres d'analyse sont régulièrement rafraîchies, le mode de glissement utilisé pour effectuer ce rafraîchissement garantissant la prise en compte de tous les clignements de paupières.

De plus, en vue de garantir une estimation viable sur chaque fenêtre d'analyse, on valide avantageusement les données d'une fenêtre d'analyse si le nombre de clignements détectés pendant la dite fenêtre d'analyse est supérieur à un seuil donné.

Par ailleurs, de façon avantageuse selon l'invention, on établit une classification des durées de clignement composée de trois classes correspondant respectivement à des clignements de courte durée « C », moyenne durée « M », et longue durée « L ».

Ainsi, à titre d'exemple, cette classification des durées de clignement peut avantageusement comporter :

- une classe courte durée « C » correspondant à des clignements de durée inférieure à une valeur de l'ordre de 150 ms à 250ms,
- une classe moyenne durée « M » correspondant à des clignements de durée supérieure à une valeur de l'ordre de 150 ms à 250ms, et inférieure à une valeur de l'ordre de 350 ms à 500 ms,
- et une classe longue durée « L » correspondant à des clignements de durée supérieure à une valeur de l'ordre de 350 ms à 500 ms.

Selon l'invention, la classification des états de vigilance comporte, quant à elle, avantageusement au moins trois classes d'état de vigilance :

- une classe « éveillé » définie par un nombre de clignements de moyenne durée inférieur à un seuil donné,
- au moins une classe intermédiaire correspondant à un état « de somnolence », définie par un nombre de clignements de moyenne durée supérieur à un seuil donné, et par un nombre de clignements de longue durée inférieur à un seuil donné,
- et une classe « endormi » définie par un nombre de clignements de longue durée supérieur à un seuil donné.

De façon avantageuse, cette classification se compose de quatre classes d'état de vigilance : la classe « éveillé », la classe « endormi », et deux classes intermédiaires « de somnolence » consistant en :

- une première classe intermédiaire correspondant à un état « un peu somnolent », définie par un nombre de clignements de moyenne durée supérieur à un seuil donné et inférieur à un seuil intermédiaire donné, et par un nombre de clignements de longue durée inférieur à un seuil donné,
- et une seconde classe intermédiaire correspondant à un état « somnolent », définie par un nombre de clignements de moyenne durée supérieur au seuil intermédiaire, et par un nombre de clignements de longue durée inférieur à un seuil donné.

Par ailleurs, de même que pour la classification des durées de clignement, le procédé d'évaluation selon l'invention consiste avantageusement à introduire un degré de progressivité dans la classification des états de vigilance, en vue de tenir compte des incertitudes et des ambiguïtés des estimations. A cet effet :

- on définit les n classes d'état de vigilance de façon que les dites classes présentent des zones frontières de transition progressive, par exemple par mise en oeuvre d'une méthode mathématique telle que la logique « floue »,
- et on délivre une information représentative de l'état de vigilance du conducteur consistant en un vecteur de n états de vigilance dont chaque composante représente le degré d'activation de l'état de vigilance correspondant.

Selon ce concept, l'état de vigilance du conducteur est donc exprimé sous la forme d'un vecteur de n états dont chaque composante indique le degré d'activation de l'état, c'est-à-dire le degré d'appartenance à la classe correspondante, chacun des dits degrés d'activation étant compris entre 0 et 1, et la somme des n degrés d'activation étant égale à 1.

Sur la base de ce principe de progressivité, l'information représentative de l'état de vigilance du conducteur consiste alors, avantageusement selon l'invention, en un vecteur de 4 états de vigilance dont chaque composante représente le degré d'activation d'un état de vigilance selon les définitions suivantes :

- degré d'activation de la classe « éveillé » = f1($\Sigma$ degrés d'appartenance à la classe moyenne durée « M », $\Sigma$ degrés d'appartenance à la classe longue durée « L »),
- degré d'activation de la classe « un peu somnolent » = f2($\Sigma$ degrés d'appartenance à la classe moyenne durée « M », $\Sigma$ degrés d'appartenance à la classe longue durée « L »),
- degré d'activation de la classe « somnolent » = f3($\Sigma$

degrés d'appartenance à la classe moyenne durée « M », Σ degrés d'appartenance à la classe longue durée « L »),

- degré d'activation de la classe « endormi » = f4(Σ degrés d'appartenance à la classe longue durée « L »).

Selon une autre spécificité de l'invention, le procédé d'évaluation vise à introduire un niveau de confiance dans le diagnostic réalisé. A cet effet, et de façon avantageuse selon l'invention :

- dans une phase préalable, on associe à chaque classe d'état de vigilance au moins une classe de durée considérée comme déterminante dans la représentation temporelle de la dite classe d'état de vigilance,
- et lors du déroulement d'une procédure d'évaluation :

  • on analyse les mouvements des deux paupières du conducteur, et on procède, pour chaque clignement, à une comparaison des deux signaux représentatifs du mouvement des deux paupières selon des critères de comparaison prédéterminés, tels que critères relatifs à la simultanéité, à l'amplitude ou aux pentes des dits signaux, de façon à déterminer, pour chaque clignement, un degré de confiance ci représentatif de la corrélation entre les deux signaux,

  • et pour chaque fenêtre d'analyse, on associe à chaque information incorporant une classe d'état de vigilance, un degré de confiance C° à associer à cette classe d'état de vigilance, tel que :

$$C° = \sum d_i.c_i \,/\, \sum d_i \text{ avec :}$$

    * di degré d'appartenance d'un clignement à chacune des classes de durée sélectionnée comme déterminante dans la représentation temporelle de la classe d'état de vigilance,
    * ci degré de confiance du clignement.

A titre d'exemple concernant la détermination des degrés de confiance, on associe, dans la phase préalable :

- à la classe d'état « éveillé », la classe de courte durée,
- à la classe d'état « un peu somnolent », le cumul des classes de courte durée et de moyenne durée,
- à la classe d'état « somnolent », la classe moyenne durée,
- et à la classe d'état « endormi », la classe longue durée.

De plus, on utilise avantageusement selon l'invention, en vue de la détermination du degré de confiance associé à chaque clignement, une combinaison de critères de comparaison relatifs à la simultanéité, à l'amplitude et aux pentes des deux signaux représentatifs du mouvement des deux paupières.

Le procédé d'évaluation selon l'invention tel que défini ci-dessus fournit une estimation instantanée de l'état de vigilance du conducteur.

Afin de compléter cette estimation instantanée, et de façon avantageuse selon l'invention, on procède régulièrement à un récapitulatif des informations délivrées lors des K dernières fenêtres d'analyse, avec K entier prédéterminé, et on réalise un lissage des données correspondantes, de façon à fournir un vecteur de n états de vigilance récapitulatif dont chaque composante consiste en une valeur récapitulative moyenne du degré d'activation de l'état de vigilance correspondant.

De plus, de façon avantageuse, on détermine également, à partir du récapitulatif des informations délivrées lors des K dernières fenêtres d'analyse, par une méthode mathématique telle que la méthode des moindres carrés, un index d'évolution représentatif de l'évolution de l'état de vigilance du conducteur durant les K dernières fenêtres d'analyse.

De telles récapitulations périodiques, d'une part, permettent d'exclure les éventuelles estimations non réalistes par une opération de lissage, et d'autre part, fournissent un index d'évolution représentatif, sur une longue période, de la tendance d'évolution de l'état de vigilance.

Afin de compléter la portée des états récapitulatifs, on intègre, en outre avantageusement les degrés de confiance dans le récapitulatif d'informations, de façon à associer un degré de confiance de valeur moyenne à chaque état de vigilance.

Le procédé d'évaluation selon l'invention ci-dessus défini est conçu pour réaliser un diagnostic sur l'état de vigilance d'un conducteur sur la base de seules informations physiologiques, sans requérir d'autres sources d'informations.

Toutefois, à des fins d'augmentation de la robustesse du procédé d'évaluation, on procède à l'introduction de niveaux de pondération lors des prises de diagnostic fondées sur l'analyse des mouvements de paupières, en intégrant avantageusement :

- d'une part, des informations dites environnementales, représentatives de conditions de conduite, telles que temps de conduite, température dans le véhicule, heure de la journée, type de chaussée (route, autoroute, urbaine...), données concernant le conducteur (âge, expérience...),
- d'autre part, des informations issues d'observations comportementales, telles que le maintien de la trajectoire du véhicule.

D'autres caractéristiques buts et avantages de l'invention ressortiront de la description détaillée qui suit en référence aux dessins annexés qui en représentent à titre d'exemple non limitatif un mode de réalisation préférentiel. Sur ces dessins :

- la figure 1 est une illustration d'un signal représentatif de mouvements de fermeture, dits clignements, d'une paupière,
- la figure 2 représente, à une échelle agrandie, la signature type d'un clignement,
- et la figure 3 est un graphique de détermination, en logique floue, de la classification des durées de clignements.

Le procédé selon l'invention vise, à titre principal, à fournir, en temps réel et en instantané, des estimations sur l'état de vigilance d'un conducteur basées sur l'observation des clignements des paupières de ce dernier.

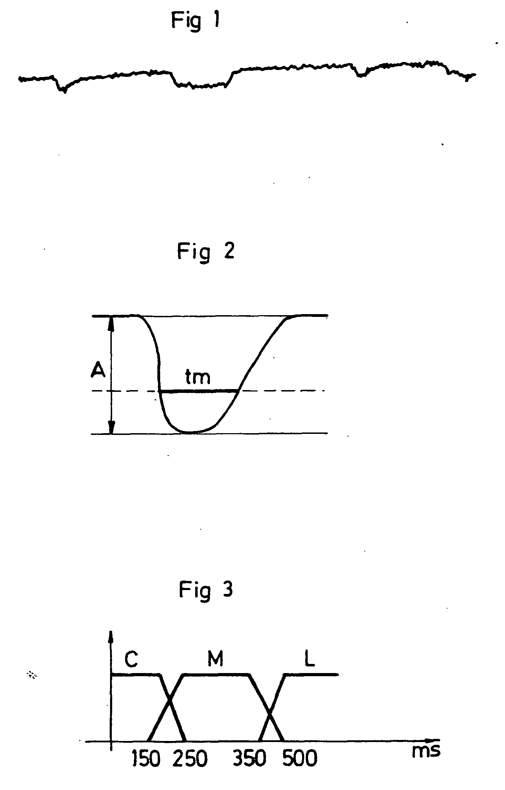

A cet effet, et de façon usuelle, la mise en oeuvre de ce procédé requiert des capteurs aptes à délivrer un signal, tel que celui représenté à la figure 1, permettant, de façon connue en soi, de détecter chaque clignement, et de déterminer pour chacun des dits clignements, tel que celui représenté à la figure 2 :

- la durée tm du clignement,
- l'amplitude A du clignement,
- et les temps de fermeture et d'ouverture définis par les pentes des fronts d'entrée et de sortie.

En premier lieu, les estimations sont fournies au terme d'intervalles de temps, dits fenêtres d'analyse, déclenchés systématiquement lors de chaque détection d'un clignement, et adaptés pour couvrir et traiter les clignements détectés sur une période de temps déterminée, par exemple de 30 secondes, précédant le déclenchement de la fenêtre d'analyse.

La première opération effectuée lors de chaque fenêtre d'analyse consiste à classer chaque clignement en fonction de sa durée en utilisant une classification des durées de clignement comportant 3 classes définissant respectivement des clignements de courte durée « C », moyenne durée « M », et longue durée « L ». De plus, ces classes consistent en des ensembles flous et présentent donc des zones frontières de transition progressive.

A titre d'exemple, et tel que représenté à la figure 3 :

- la classe courte durée « C » couvre les durées de clignements comprises entre 0 ms et 250 ms,
- la classe moyenne durée « M » couvre les durées de clignements comprises entre 150 ms et 500 ms,
- et la classe longue durée « L » couvre les durées de clignements supérieures à 350 ms.

De plus, selon le principe de la logique floue, et tel que représenté à la figure 3, les clignements dont la durée correspond à une zone frontière entre deux classes appartiennent simultanément à ces deux classes, le degré d'appartenance à chacune des dites deux classes étant inférieur à 1, et la somme des dits deux degrés d'appartenance étant égale à 1.

Cette classification conduit ainsi à définir chaque clignement par un vecteur (3, 1) dont les trois composantes correspondent respectivement au degré d'appartenance du dit clignement à chacune des trois classes de durée.

Après la définition de tous les clignements détectés lors d'une fenêtre d'analyse, l'opération suivante consiste à déterminer un vecteur cumulé de durée dont chaque composante consiste en la somme $\Sigma C$, $\Sigma M$, $\Sigma L$ des composantes de même ligne des vecteurs définissant les dits clignements.

Selon le principe de l'invention, ce vecteur cumulé est destiné à servir de base à la détermination de l'état de vigilance pour la fenêtre d'analyse concernée, moyennant l'utilisation d'une classification des états de vigilance comportant les quatre classes suivantes définies chacune ci-dessous avec les règles déterminant l'appartenance à la dite classe :

- une classe correspondant à un état « éveillé », répondant aux conditions d'appartenance suivantes : $\Sigma L \leq N1$, et $\Sigma M \leq N2$,
- une classe correspondant à un état « un peu somnolent », répondant aux conditions d'appartenance suivantes : $\Sigma L \leq N3$, et $N2 < \Sigma M \leq N4$,
- une classe correspondant à un état « somnolent », répondant aux conditions d'appartenance suivantes : $\Sigma L \leq N3$, et $N4 < \Sigma M \leq N5$,
- et une classe correspondant à un état « endormi », répondant aux conditions d'appartenance suivantes : $\Sigma L > N3$,
- les nombres Ni ci-dessus étant tels que $Ni < Ni+1$

De plus, tel que précédemment, ces classes d'état de vigilance sont définis de façon à consister en des ensembles flous de sorte que chaque état de vigilance est défini par un vecteur de 4 états de vigilance dont les composantes représentent les degrés d'activation respectifs des quatre états de vigilance, à savoir :

- le degré d'activation de la classe « éveillé » = f1($\Sigma$ degrés d'appartenance à la classe moyenne durée « M », $\Sigma$ degrés d'appartenance à la classe longue durée « L »),
- le degré d'activation de la classe « un peu somnolent » = f2($\Sigma$ degrés d'appartenance à la classe moyenne durée « M », $\Sigma$ degrés d'appartenance à la classe longue durée « L »),
- le degré d'activation de la classe « somnolent » = f3 ($\Sigma$ degrés d'appartenance à la classe moyenne durée « M », $\Sigma$ degrés d'appartenance à la classe longue durée « L »),
- et le degré d'activation de la classe « endormi » = f4

($\Sigma$ degrés d'appartenance à la classe longue durée « L »).

En outre, plusieurs techniques, connues en elles-mêmes, peuvent être utilisées pour représenter les fonctions fi précitées.

Ainsi, une première technique peut consister à définir des ensembles flous tridimensionnels conçus pour permettre d'obtenir directement les divers degrés d'activation sans utilisation de règles de logique floue.

Une deuxième technique plus classique peut également consister à définir des ensembles flous mono dimensionnels pour décrire chacune des entrées $\Sigma M$, $\Sigma L$, et à élaborer des règles de logique floue pour combiner ces entrées et en déduire les divers degrés d'activation.

Quelle que soit la technique mise en oeuvre, le procédé d'évaluation selon l'invention conduit donc à fournir, pour chaque fenêtre d'analyse, un état de vigilance se présentant sous la forme d'un vecteur de quatre états dont chaque composante indique le degré d'activation de l'état.

Un exemple d'état de vigilance fourni conformément à l'invention est donné ci-dessous à titre d'illustration.

| ETAT | DEGRE D'ACTIVATION |
|------|--------------------|
| Eveillé | 0,2 |
| Un peu somnolent | 0,4 |
| Somnolent | 0,4 |
| Endormi | 0 |

Une autre spécificité de l'invention consiste à associer un degré de confiance à chaque état de vigilance fourni.

A cet effet, on attribue, en premier lieu, un degré de confiance « ci » à chaque mesure de durée d'un clignement. Pour ce faire, on analyse les mouvements des deux paupières du conducteur, et on procède, pour chaque clignement, à une comparaison des deux signaux représentatifs du mouvement des deux paupières basée sur des critères de comparaison prédéterminés tels que :

- critères de simultanéité : comparaison de la durée des signaux et des instants des tops de début et de fin des dits signaux,
- critères d'amplitude : comparaison des amplitudes des signaux,
- critères de pentes : comparaison des pentes respectives des fronts d'entrée et de sortie des signaux.

Chaque degré de confiance ci est ainsi avantageusement déterminé à partir d'une combinaison des différents critères de comparaison, en attribuant éventuellement des poids différents aux divers critères. (A titre d'exemple, de façon usuelle, le critère de simultanéité est ainsi sélectionné comme le critère prépondérant).

Les critères de confiance ci calculés selon ce principe sont donc tels que $ci = (a.Csimul + b.Camp + c.Cpente) / (a + b + c)$ avec :

- $0 \le Csimul, Camp, Cpente \le 1$
- et $0 \le a, b, c \le 1$.

Une autre technique de détermination des degrés de confiance ci peut également consister à utiliser la méthode de la logique floue.

La finalité des calculs de ces degrés de confiance consiste à associer, à chaque état de vigilance, des degrés de confiance C° tels que :

C° « éveillé » = $\Sigma di.ci / \Sigma di$, pour tous les clignements de courte durée,

C° « un peu somnolent » = $\Sigma di.ci / \Sigma di$, pour tous les clignements de courte durée et tous les clignements de moyenne durée,

C° « somnolent » = $\Sigma di.ci / \Sigma di$, pour tous les clignements de moyenne durée,

et C° « endormi » = $\Sigma di.ci / \Sigma di$, pour tous les clignements de longue durée.

En outre, dans ces expressions :

- di représente le degré d'appartenance d'un clignement à la classe de durée visée,
- et ci le degré de confiance du clignement.

Une autre spécificité de l'invention consiste à réaliser régulièrement un récapitulatif ou historique des informations délivrées lors des K dernières fenêtres d'analyse, avec K entier prédéterminé, et à effectuer un lissage des données correspondantes de façon à fournir un vecteur de 4 états de vigilance récapitulatif dont chaque composante consiste en une valeur récapitulative moyenne du degré d'activation de l'état de vigilance correspondant.

De plus, on intègre également les degrés de confiance dans le récapitulatif d'informations, de façon à associer un degré de confiance de valeur moyenne à chaque état de vigilance.

Il est noter que selon l'invention, les techniques de lissage peuvent consister soit en un simple calcul arithmétique de la valeur moyenne des données considérées, soit en des méthodes de lissage plus complexes de tout type connu en soi.

Les récapitulatifs ont aussi pour fonction première d'exclure, par une opération de lissage, les éventuelles estimations non réalistes.

De plus, ces récapitulatifs ont pour but de permettre de calculer un index d'évolution représentatif de l'évolution de l'état de vigilance du conducteur durant les K dernières fenêtres d'analyse, et donc de la tendance d'évolution de l'état de vigilance.

Cet index d'évolution est calculé selon la méthode usuelle des moindres carrés qui fournit, en fait, une approximation de la pente d'interpolation passant par tous les différents points.

Le procédé d'évaluation ci-dessus décrit permet de réaliser en ligne un diagnostic de la baisse de vigilance d'un conducteur à partir d'informations d'ordre physiologique.

Il peut toutefois être intéressant de parfaire la fiabilité de ce diagnostic en pondérant ce dernier par des informations complémentaires visant à aboutir à un renforcement ou à une relaxation de la décision.

A ce titre, et en vue d'introduire des niveaux de pondération lors des prises de diagnostic fondées sur l'analyse des mouvements de paupières, on intègre :

- des informations environnementales représentatives de conditions de conduite, telles que temps de conduite, température dans le véhicule, heure de la journée, type de chaussée (route, autoroute, urbaine...), données concernant le conducteur (âge, expérience...),
- et des informations issues d'observations comportementales, telles que l'observation de la trajectoire du véhicule.

**Revendications**

1. Procédé d'évaluation de l'état de vigilance d'un conducteur de véhicule, consistant à analyser les mouvements d'au moins une paupière du dit conducteur de façon à détecter chaque fermeture de la dite paupière, dite clignement, et à fournir une information représentative de la durée du dit clignement, le dit procédé d'évaluation consistant :

   - dans une phase préalable :

     • à établir une classification des durées de clignement composée de m classes délimitant m plages contiguës de valeurs de durées de clignements, consistant en au moins deux classes correspondant respectivement à des clignements dits de moyenne durée « M », et de longue durée « L », les dites classes étant adaptées pour présenter des zones frontières de transition progressive, définies par exemple par mise en oeuvre d'une méthode mathématique telle que la logique « floue »,
     • et à établir une classification d'états de vigilance composée de n classes d'état de vigilance, avec n ≥ 2, comportant :

       * une classe correspondant à un état « éveillé », définie par un nombre de clignements de moyenne durée inférieur à un seuil donné, et/ou un nombre de clignements de longue durée inférieur à un seuil donné,
       * et une classe correspondant à un état « endormi », définie par un nombre de clignements de moyenne durée supérieur à un seuil donné, et/ou un nombre de clignements de longue durée supérieur à un seuil donné,

   - et lors du déroulement d'une procédure d'évaluation :

     • à associer à chaque clignement un vecteur de durée (m, 1) dont chaque composante représente le degré d'appartenance du dit clignement à une des m classes de durée prédéfinies,
     • à définir des fenêtres temporelles d'analyse consistant en des intervalles de temps au terme de chacun desquels on calcule un vecteur cumulé de durée dont chaque composante consiste en la somme $\Sigma M$, $\Sigma L$ des composantes de même ligne des vecteurs de durée correspondant aux clignements détectés pendant la fenêtre d'analyse,
     • et à déduire de la comparaison des valeurs calculées $\Sigma M$ et $\Sigma L$ avec les valeurs seuils correspondantes de la classification des états de vigilance, une information représentative de l'état de vigilance du conducteur.

2. Procédé d'évaluation selon la revendication 1 **caractérisé en ce que** :

     • on définit les n classes d'état de vigilance de façon que les dites classes présentent des zones frontières de transition progressive, par exemple par mise en oeuvre d'une méthode mathématique telle que la logique « floue »,
     • on délivre une information représentative de l'état de vigilance du conducteur consistant en un vecteur de n états de vigilance dont chaque composante représente le degré d'activation de l'état de vigilance correspondant.

3. Procédé d'évaluation selon l'une des revendications 1 ou 2 **caractérisé en ce que** l'on établit une classification des durées de clignement composée de trois classes correspondant respectivement à des clignements de courte durée « C », moyenne durée « M », et longue durée « L ».

4. Procédé d'évaluation selon l'une des revendications précédentes **caractérisé en ce que** la classification des états de vigilance comporte au moins trois classes d'état de vigilance :

- une classe « éveillé » définie par un nombre de clignements de moyenne durée inférieur à un seuil donné,

- au moins une classe intermédiaire correspondant à un état « de somnolence », définie par un nombre de clignements de moyenne durée supérieur à un seuil donné, et par un nombre de clignements de longue durée inférieur à un seuil donné,

- et une classe « endormi » définie par un nombre de clignements de longue durée supérieur à un seuil donné.

5. Procédé d'évaluation selon la revendication 4 **caractérisé en ce que** la classification des états de vigilance comporte quatre classes d'état de vigilance : la classe « éveillé », la classe « endormi », et deux classes intermédiaires « de somnolence » consistant en :

- une première classe intermédiaire correspondant à un état « un peu somnolent », définie par un nombre de clignements de moyenne durée supérieur à un seuil donné et inférieur à un seuil intermédiaire donné, et par un nombre de clignements de longue durée inférieur à un seuil donné,

- et une seconde classe intermédiaire correspondant à un état «somnolent», définie par un nombre de clignements de moyenne durée supérieur au seuil intermédiaire, et par un nombre de clignements de longue durée inférieur à un seuil donné.

6. Procédé d'évaluation selon les revendications 2 et 5 prises ensemble **caractérisé en ce que** l'information représentative de l'état de vigilance du conducteur consiste en un vecteur de 4 états de vigilance dont chaque composante représente le degré d'activation d'un état de vigilance selon les définitions suivantes :

- degré d'activation de la classe « éveillé » = f1 ($\Sigma$ degrés d'appar-tenance à la classe moyenne durée « M », $\Sigma$ degrés d'appartenance à la classe longue durée « L »),

- degré d'activation de la classe « un peu somnolent » = f2($\Sigma$ degrés d'appartenance à la classe moyenne durée « M », $\Sigma$ degrés d'appartenance à la classe longue durée « L »),

- degré d'activation de la classe « somnolent » = f3($\Sigma$ degrés d'appartenance à la classe moyenne durée « M », $\Sigma$ degrés d'appartenance à la classe longue durée « L »),

- degré d'activation de la classe « endormi » = f4($\Sigma$ degrés d'appartenance à la classe longue durée « L »).

7. Procédé d'évaluation selon l'une des revendications précédentes **caractérisé en ce que** :

- dans une phase préalable, on associe à chaque classe d'état de vigilance au moins une classe de durée considérée comme déterminante dans la représentation temporelle de la dite classe d'état de vigilance,

- et lors du déroulement d'une procédure d'évaluation :

• on analyse les mouvements des deux paupières du conducteur, et on procède, pour chaque clignement, à une comparaison des deux signaux représentatifs du mouvement des deux paupières selon des critères de comparaison prédéterminés, tels que critères relatifs à la simultanéité, à l'amplitude ou aux pentes des dits signaux, de façon à déterminer, pour chaque clignement, un degré de confiance ci représentatif de la corrélation entre les deux signaux,

• et pour chaque fenêtre d'analyse, on associe à chaque information incorporant une classe d'état de vigilance, un degré de confiance C° à associer à cette classe d'état de vigilance, tel que :

$$C° = \sum d_i.c_i \; / \; \sum d_i \quad \text{avec :}$$

\* di degré d'appartenance d'un clignement à chacune des classes de durée sélectionnée comme déterminante dans la représentation temporelle de la classe d'état de vigilance,

\* ci degré de confiance du clignement.

8. Procédé d'évaluation selon la revendication 7 **caractérisé en ce que** l'on utilise, en vue de la détermination du degré de confiance associé à chaque clignement, une combinaison de critères de comparaison relatifs à la simultanéité, à l'amplitude et aux pentes des deux signaux représentatifs du mouvement des deux paupières.

9. Procédé d'évaluation selon la revendication 2 **caractérisé en ce que** l'on procède régulièrement à un récapitulatif des informations délivrées lors des K dernières fenêtres d'analyse, avec K entier prédéterminé, et **en ce que** l'on réalise un lissage des données correspondantes de façon à fournir un vecteur de n états de vigilance récapitulatif dont chaque composante consiste en une valeur récapitulative moyenne du degré d'activation de l'état de vigilance correspondant.

10. Procédé d'évaluation selon la revendication 9 **ca-**

**ractérisé en ce que** l'on détermine, à partir du récapitulatif des informations délivrées lors des K dernières fenêtres d'analyse, par une méthode mathématique telle que la méthode des moindres carrés, un index d'évolution représentatif de l'évolution de l'état de vigilance du conducteur durant les K dernières fenêtres d'analyse.

11. Procédé d'évaluation selon l'une des revendications 9 ou 10 et la revendication 7 prises ensemble, **caractérisé en ce que** l'on intègre les degrés de confiance dans le récapitulatif d'informations, de façon à associer un degré de confiance de valeur moyenne à chaque état de vigilance.

12. Procédé d'évaluation selon l'une des revendications précédentes **caractérisé en ce qu'**il consiste, en outre, à intégrer des informations dites environnementales, représentatives de conditions de conduite, telles que temps de conduite, température dans le véhicule, heure de la journée, type de chaussée (route, autoroute, urbaine...), données concernant le conducteur (âge, expérience...), en vue d'introduire des niveaux de pondération lors des prises de diagnostic fondées sur l'analyse des mouvements de paupières.

13. Procédé d'évaluation selon l'une des revendications précédentes **caractérisé en ce qu'**il consiste, en outre, à intégrer des informations issues d'observations comportementales, telles que l'observation de la trajectoire du véhicule, en vue d'introduire des niveaux de pondération lors des prises de diagnostic fondées sur l'analyse des mouvements de paupières.

14. Procédé d'évaluation selon l'une des revendications précédentes **caractérisé en ce que** l'on déclenche l'ouverture d'une fenêtre d'analyse lors de chaque détection d'un clignement, chaque fenêtre d'analyse ouverte couvrant une période de temps déterminée précédant le dit déclenchement.

15. Procédé d'évaluation selon l'une des revendications précédentes **caractérisé en ce que** l'on valide les données d'une fenêtre d'analyse si le nombre de clignements détectés pendant la dite fenêtre d'analyse est supérieur à un seuil donné.

**Claims**

1. Method of assessing the state of alertness of a vehicle driver, consisting of analysing the movements of at least one of said driver's eyelids so as to detect each closure of said eyelid, known as a blink, and of providing information representative of the duration of said blink, said method of assessment consisting:

- in a preliminary phase:

    • of establishing a classification of blink durations comprising m categories delimiting m contiguous ranges of blink duration values, consisting of at least two categories corresponding to blinks said to be of medium duration "M" and of long duration "L" respectively, said categories being suitable for presenting progressive transition border zones, defined for example by implementing a mathematical method such as "fuzzy logic",
    • and of establishing a classification of states of alertness comprising n categories of states of alertness, with n≥2, comprising:

        • a category corresponding to an "alert" state, defined by a number of blinks of medium duration below a given threshold, and/or a number of blinks of long duration below a given threshold,
        • and a category corresponding to a "sleepy" state, defined by a number of blinks of medium duration above a given threshold, and/or a number of blinks of long duration above a given threshold,

- and during the course of an assessment procedure:

    • of associating with each blink a duration vector (m,1), of which each component represents said blink's degree of membership to one of the m predefined categories of duration,
    • of defining temporal analysis windows consisting of time intervals, after each of which a cumulative duration vector is calculated, of which each component consists of the sum ΣM, ΣL of the components on the same line in the duration vectors corresponding to the blinks detected during the analysis window,
    • and of deducing, from the comparison of the calculated values ΣM and ΣL against their corresponding threshold values in the classification of states of alertness, information representative of the state of alertness of the driver.

2. Method of assessment according to claim 1, **characterised in that**:

    • the n categories of state of alertness are defined so that said categories present progressive transition border zones, for example by implementing a mathematical method, such as "fuzzy" logic,
• information is delivered that is representative of the driver's state of alertness, consisting of a vector of n states of alertness, of which each component represents the degree of activation of the corresponding state of alertness.

3. Method of assessment according to claims 1 or 2, **characterised in that** a classification of blink durations is established, comprising three categories corresponding to blinks of short duration "C", medium duration "M" and long duration "L" respectively.

4. Method of assessment according to any of the preceding claims **characterised in that** the classification of states of alertness comprises at least three categories of states of alertness:

   - an "alert" category, defined by a number of blinks of medium duration below a given threshold,
   - at least one intermediate category corresponding to a state of "drowsiness", defined by a number of blinks of medium duration above a given threshold, and by a number of blinks of long duration below a given threshold,
   - and a "sleepy" category, defined by a number of blinks of long duration above a given threshold.

5. Method of assessment according to claim 4, **characterised in that** the classification of states of alertness comprises four categories of state of alertness: the "alert" category, the "sleepy" category and two intermediate "drowsy" categories, consisting of:

   - a first intermediate category corresponding to a "slightly drowsy" state, defined by a number of blinks of medium duration above a given threshold and below a given intermediate threshold, and by a number of blinks of long duration below a given threshold,
   - and a second intermediate category corresponding to a "drowsy" state, defined by a number of blinks of medium duration above the intermediate threshold, and by a number of blinks of long duration below a given threshold.

6. Method of assessment according to claims 2 and 5 taken together, **characterised in that** the information representative of the driver's state of alertness consists of a vector of 4 states of alertness, of which each component represents the degree of activation of a state of alertness according to the following definitions:

   - degree of activation of the "alert" category = f1

($\Sigma$ degrees of membership to the medium duration category "M", $\Sigma$ degrees of membership to the long duration category "L"),
   - degree of activation of the "slightly drowsy" state = f2 ($\Sigma$ degrees of membership to the medium duration category "M", $\Sigma$ degrees of membership to the long duration category "L"),
   - degree of activation of the "drowsy" category = f3 ($\Sigma$ degrees of membership to the medium duration category "M", $\Sigma$ degrees of membership to the long duration category "L")
   - degree of activation of the "sleepy" category = f4 ($\Sigma$ degrees of membership to the long duration category "L").

7. Method of assessment according to any of the preceding claims, **characterised in that**:

   - in a preliminary phase, each category of state of alertness is associated with at least one category of duration considered to be a determining factor in the temporal representation of said category of state of alertness,
   - and during the course of an assessment procedure:

      • the movements of both of the driver's eyelids are analysed and, for each blink, a comparison is made of the two signals representative of the movement of the two eyelids according to predetermined comparison criteria, such as criteria relating to simultaneity, amplitude or the slopes of said signals, so as to determine for each blink a degree of confidence ci representative of the correlation between the two signals,
      • and for each analysis window, a degree of confidence C° is associated with each piece of information incorporating a category of state of alertness, to be associated with this category of state of alertness, such as:

$$C° = \Sigma \ di.ci/\Sigma \ di \ \text{with:}$$

      • di degree of a blink's membership to each of the categories of duration selected as being determining factors in the temporal representation of the category of state of alertness,
      • ci degree of confidence for the blink.

8. Method of assessment according to claim 7, **characterised in that**, with a view to determining the degree of confidence associated with each blink, a combination of comparison criteria is used relating to the simultaneity, the amplitude and the slopes of the two signals representative of the movements of the two eyelids.

9. Method of assessment according to claim 2, **characterised in that** a regular summary is made of the information delivered during the last K analysis windows, with integer K predetermined, and **in that** smoothing of the corresponding data is carried out so as to provide a summary vector of n states of alertness, of which each component consists of a mean summary value for the degree of activation of the corresponding state of alertness.

10. Method of assessment according to claim 9, **characterised in that**, using the summary of information delivered during the last K analysis windows, and via a mathematical method such as the least squares method, a progress index is determined, representative of the development of the driver's state of alertness during the last K analysis windows.

11. Method of assessment according to claims 9 or 10 and claim 7 taken together, **characterised in that** degrees of confidence are integrated into the information summary, so as to associate a mean value degree of confidence with each state of alertness.

12. Method of assessment according to any of the preceding claims, **characterised in that** it further consists of incorporating information said to be environmental, representative of the driving conditions, such as driving time, temperature inside the vehicle, time of day, type of road (main road, motorway, urban road etc.), data relating to the driver (age, experience etc.), with a view to introducing weighting levels during diagnostic procedures based on the analysis of eyelid movements.

13. Method of assessment according to any of the preceding claims, **characterised in that** it further consists of integrating information derived from behavioural observations, such as maintenance of the vehicle's path of travel, with a view to introducing weighting levels during diagnostic procedures based on the analysis of eyelid movements.

14. Method of assessment according to any of the preceding claims, **characterised in that** the opening of an analysis window is triggered each time a blink is detected, each analysis window opened covering a specified period of time preceding said triggering process.

15. Method of assessment according to any of the preceding claims, **characterised in that** the data of an analysis window is validated if the number of blinks detected during said analysis window is above a given threshold.

**Patentansprüche**

1. Verfahren zur Bewertung des Aufmerksamkeitszustands eines Fahrzeugfahrers, das darin besteht, die Bewegungen von zumindest einem Augenlid des Fahrers zu analysieren, so dass jede Schließung des Augenlids, der so genannte Lidschlag, erfasst wird, und eine Information zu liefern, die für die Dauer des Lidschlags bezeichnend ist, wobei das Bewertungsverfahren darin besteht:

   - in einer Vorphase:

      • eine Klassifizierung der Lidschlagdauern zu erstellen, die m Kategorien umfasst, die m aneinander grenzende Bereiche von Lidschlagdauerwerten eingrenzen, und die sich aus mindestens zwei Kategorien zusammensetzt, die jeweils Lidschlägen von so genannter mittlerer Dauer "M" und langer Dauer "L" entsprechen, wobei die Kategorien angepasst sind, um progressive Übergangsgrenzbereiche aufzuweisen, die beispielsweise durch Anwenden einer mathematischen Methode wie etwa der Fuzzy-Logik definiert werden,
      • und eine Klassifizierung von Aufmerksamkeitszuständen zu erstellen, die n Kategorien von Aufmerksamkeitszuständen umfasst, mit n ≥ 2, und die Folgendes umfasst:

         * eine Kategorie, die einem Zustand "wach" entspricht und die durch eine Anzahl von Lidschlägen mittlerer Dauer definiert wird, die niedriger ist als ein gegebener Grenzwert, und/oder durch eine Anzahl von Lidschlägen langer Dauer, die niedriger ist als ein gegebener Grenzwert,
         * und eine Kategorie, die einem Zustand "eingeschlafen" entspricht und die durch eine Anzahl von Lidschlägen mittlerer Dauer definiert wird, die höher ist als ein gegebener Grenzwert, und/oder durch eine Anzahl von Lidschlägen langer Dauer, die höher ist als ein gegebener Grenzwert,

   - und beim Ablauf eines Bewertungsverfahrens:

      • jedem Lidschlag einen Dauervektor (m, 1) zuzuordnen, von dem jede Komponente den Zugehörigkeitsgrad des Lidschlags zu einer der m vordefinierten Dauerkategorien repräsentiert,
      • Analysezeitfenster zu definieren, die aus Zeitintervallen bestehen, wobei am Ende jedes dieser Intervalle ein addierter Dauervektor berechnet wird, von dem jede Komponente aus der Summe ΣM, ΣL der Dauervektorkomponenten derselben Linie besteht, die den Lidschlägen, die während des Analysefensters erfasst werden, entsprechen,

• und aus dem Vergleich der berechneten Werte ΣM und ΣL mit den Grenzwerten, die der Klassifizierung der Aufmerksamkeitszustände entsprechen, eine Information abzuleiten, die für den Aufmerksamkeitszustand des Fahrers bezeichnend ist.

2. Bewertungsverfahren nach Anspruch 1, **dadurch gekennzeichnet, dass**:

> • die n Aufmerksamkeitszustandskategorien so definiert werden, dass die besagten Kategorien progressive Übergangsgrenzbereiche aufweisen, beispielsweise durch Anwenden einer mathematischen Methode wie etwa der Fuzzy-Logik,
> • eine Information ausgegeben wird, die für den Aufmerksamkeitszustand des Fahrers bezeichnend ist und die in einem Vektor von n Aufmerksamkeitszuständen besteht, von dem jede Komponente den Aktivierungsgrad des entsprechenden Aufmerksamkeitszustands repräsentiert.

3. Bewertungsverfahren nach einem der Ansprüche 1 oder 2, **dadurch gekennzeichnet, dass** eine Klassifizierung der Lidschlagdauern erstellt wird, die drei Kategorien umfasst, die jeweils Lidschlägen kurzer Dauer "C", mittlerer Dauer "M" und langer Dauer "L" entsprechen.

4. Bewertungsverfahren nach einem der vorhergehenden Ansprüche, **dadurch gekennzeichnet, dass** die Klassifizierung der Aufmerksamkeitszustände zumindest drei Aufmerksamkeitszustandskategorien umfasst:

> - eine Kategorie "wach", die durch eine Anzahl von Lidschlägen mittlerer Dauer definiert wird, die niedriger ist als ein gegebener Grenzwert,
> - zumindest eine Zwischenkategorie, die einem "schläfrigen" Zustand entspricht und die durch eine Anzahl von Lidschlägen mittlerer Dauer definiert wird, die höher ist als ein gegebener Grenzwert, und durch eine Anzahl von Lidschlägen langer Dauer, die niedriger ist als ein gegebener Grenzwert,
> - und eine Kategorie "eingeschlafen", die durch eine Anzahl von Lidschlägen langer Dauer definiert wird, die höher ist als ein gegebener Grenzwert.

5. Bewertungsverfahren nach Anspruch 4, **dadurch gekennzeichnet, dass** die Klassifizierung der Aufmerksamkeitszustände vier Aufmerksamkeitszustandskategorien umfasst: die Kategorie "wach", die Kategorie "eingeschlafen" und zwei Zwischenkategorien "schläfrig", die in Folgendem bestehen:

> - einer ersten Zwischenkategorie, die einem Zustand "etwas schläfrig" entspricht und die durch eine Anzahl von Lidschlägen mittlerer Dauer definiert wird, die höher ist als ein gegebener Grenzwert und niedriger als ein gegebener Zwischengrenzwert, und durch eine Anzahl von Lidschlägen langer Dauer, die niedriger ist als ein gegebener Grenzwert,
> - und einer zweiten Zwischenkategorie, die einem Zustand "schläfrig" entspricht und die durch eine Anzahl von Lidschlägen mittlerer Dauer definiert wird, die höher ist als der Zwischengrenzwert, und durch eine Anzahl von Lidschlägen langer Dauer, die niedriger ist als ein gegebener Grenzwert.

6. Bewertungsverfahren nach den zusammengenommenen Ansprüchen 2 und 5, **dadurch gekennzeichnet, dass** die Information, die für den Aufmerksamkeitszustand des Fahrers bezeichnend ist, in einem Vektor von 4 Aufmerksamkeitszuständen besteht, von dem jede Komponente den Aktivierungsgrad eines Aufmerksamkeitszustands gemäß den folgenden Definitionen repräsentiert:

> - Aktivierungsgrad der Kategorie "wach" = f1 (Σ Zugehörigkeitsgrade zur Kategorie mittlerer Dauer "M", Σ Zugehörigkeitsgrade zur Kategorie langer Dauer "L")
> - Aktivierungsgrad der Kategorie "etwas schläfrig" = f2 (Σ Zugehörigkeitsgrade zur Kategorie mittlerer Dauer "M", Σ Zugehörigkeitsgrade zur Kategorie langer Dauer "L")
> - Aktivierungsgrad der Kategorie "schläfrig" - f3 (Σ Zugehörigkeitsgrade zur Kategorie mittlerer Dauer "M", Σ Zugehörigkeitsgrade zur Kategorie langer Dauer "L")
> - Aktivierungsgrad der Kategorie "eingeschlafen" = f4 ( Σ Zugehörigkeitsgrade zur Kategorie langer Dauer "L").

7. Bewertungsverfahren nach einem der vorhergehenden Ansprüche, **dadurch gekennzeichnet, dass**:

> - in einer Vorphase jeder Aufmerksamkeitszustandskategorie zumindest eine Dauerkategorie zugeordnet wird, die als vorherrschend in der zeitlichen Darstellung der besagten Aufmerksamkeitszustandskategorie betrachtet wird,
> - und beim Ablauf eines Bewertungsverfahrens:

• die Bewegungen beider Augenlider des Fahrers analysiert werden und für jeden Lidschlag ein Vergleich der zwei Signale vorgenommen wird, die für die Bewegung der beiden Augenlider bezeichnend sind, gemäß im Voraus festgelegter Vergleichskriterien, wie etwa Kriterien in Bezug auf die Gleichzeitigkeit, auf die Amplitude oder auf die Steigungen der Signale, so dass für jeden Lidschlag ein Vertrauensgrad ci bestimmt werden kann, der für die Korrelation zwischen den beiden Signalen bezeichnend ist,

• und für jedes Analysefenster jeder Information, die eine Aufmerksamkeitszustandskategorie beinhaltet, ein Vertrauensgrad C° zugeordnet wird, der dieser Aufmerksamkeitszustandskategorie zuzuordnen ist, wie etwa:

$$C° = \sum di.ci\ /\ \sum di \text{ mit:}$$

* di als Zugehörigkeitsgrad eines Lidschlags zu jeder der Dauerkategorien, die als vorherrschend in der zeitlichen Darstellung der Aufmerksamkeitszustandskategorie ausgewählt werden,
* ci als Vertrauensgrad des Lidschlags.

8. Bewertungsverfahren nach Anspruch 7, **dadurch gekennzeichnet, dass** im Hinblick auf die Bestimmung des Vertrauensgrades, der jedem Lidschlag zugeordnet wird, eine Kombination von Vergleichskriterien verwendet wird, die sich auf die Gleichzeitigkeit, auf die Amplitude und auf die Steigungen der beiden Signale beziehen, die für die Bewegung der beiden Augenlider bezeichnend sind.

9. Bewertungsverfahren nach Anspruch 2, **dadurch gekennzeichnet, dass** regelmäßig eine Zusammenfassung der Informationen erstellt wird, die im Laufe der K letzten Analysefenster ausgegeben wurden, mit K als im Voraus festgelegte Ganzzahl, und dass eine Glättung der entsprechenden Daten durchgeführt wird, um einen zusammenfassenden Vektor von n Aufmerksamkeitszuständen bereitstellen zu können, von dem jede Komponente in einem zusammenfassenden Durchschnittswert des Aktivierungsgrades des entsprechenden Aufmerksamkeitszustands besteht.

10. Bewertungsverfahren nach Anspruch 9, **dadurch gekennzeichnet, dass** ausgehend von der Zusammenfassung der Informationen, die im Laufe der K letzten Analysefenster ausgegeben wurden, mittels einer mathematischen Methode, wie etwa der Methode der kleinsten Quadrate, ein Entwicklungsindex bestimmt wird, der für die Entwicklung des Aufmerksamkeitszustands des Fahrers während der K letzten Analysefenster bezeichnend ist.

11. Bewertungsverfahren nach einem der Ansprüche 9 oder 10, zusammengenommen mit dem Anspruch 7, **dadurch gekennzeichnet, dass** die Vertrauensgrade in die Informationszusammenfassung einbezogen werden, so dass jedem Aufmerksamkeitszustand ein Vertrauensgrad von durchschnittlichem Wert zugeordnet wird.

12. Bewertungsverfahren nach einem der vorhergehenden Ansprüche, **dadurch gekennzeichnet, dass** es außerdem darin besteht, so genannte Umgebungsinformationen einzubeziehen, die für die Fahrbedingungen bezeichnend sind, wie etwa Fahrdauer, Temperatur im Fahrzeug, Tageszeit, Straßenart (Landstraße, Autobahn, Stadtverkehr...), fahrerbezogene Daten (Alter, Erfahrung,...), um Gewichtungsstufen bei den Diagnosestellungen, die auf der Analyse der Augenlidbewegungen basieren, einzuführen.

13. Bewertungsverfahren nach einem der vorhergehenden Ansprüche, **dadurch gekennzeichnet, dass** es außerdem darin besteht, Informationen einzubeziehen, die aus Verhaltensbeobachtungen hervorgehen, wie etwa die Einhaltung der Spur des Fahrzeugs, um Gewichtungsstufen bei den Diagnosestellungen, die auf der Analyse der Augenlidbewegungen basieren, einzuführen.

14. Bewertungsverfahren nach einem der vorhergehenden Ansprüche, **dadurch gekennzeichnet, dass** die Öffnung eines Analysefensters bei jeder Erfassung eines Lidschlags ausgelöst wird, wobei jedes offene Analysefenster eine bestimmte Zeitdauer abdeckt, die dem Auslösen vorausgeht.

15. Bewertungsverfahren nach einem der vorhergehenden Ansprüche, **dadurch gekennzeichnet, dass** die Daten eines Analysefensters validiert werden, wenn die Anzahl der Lidschläge, die während des Analysefensters erfasst werden, höher als ein gegebener Grenzwert ist.

## Fig 1

## Fig 2

## Fig 3

**EP 1 788 536 B1**

**RÉFÉRENCES CITÉES DANS LA DESCRIPTION**

**Documents brevets cités dans la description**

- US 6070098 A